# EUROPEAN PATENT SPECIFICATION

(11) **EP 1 220 848 B1**
(45) Date of publication and mention of the grant of the patent: **19.11.2003**
(21) Application number: 00963380.1
(22) Date of filing: 12.09.2000
(51) Int. Cl.: C07D 307/68, C07D 409/12, C07D 405/12, A61K 31/506, A61K 31/4402, A61K 31/4406, A61K 31/381, A61P 31/18

(54) **N-ARYMETHYLTHIOANILIDE COMPOUNDS USEFUL FOR THE INHIBITION OF THE REPLICATION OF HIV**
N-ARYLMETHYLTHIOANILID-DERIVATE FÜR DIE HEMMUNG DER HIV-REPLIKATION
COMPOSES DE N-ARYMETHYLTHIOANILIDE UTILES POUR INHIBER LA REPLICATION DU VIH

(30) Priority: 17.09.1999 US 398649
(43) Date of publication of application: 10.07.2002
(73) Proprietor: Uniroyal Chemical Company, Inc., Middlebury, CT 06749 (US); Crompton Co./Cie, Elmira, Ontario N3B 3A3 (CA)
(72) Inventor: BROUWER, Walter, Gerhard, Guelph, Ontario N1G 2C2 (CA); OSIKA, Ewa, Maria, Kitchener, Ontario N2P 1B9 (CA)
(74) Representative: Spott, Gottfried, Dr.
(86) International application number: PCT/US00/24986
(87) International publication number: WO 01/019811

(56) References cited:
- WO-A-97/19940
- US-A- 6 017 947

## Description

### Field of the Invention

This invention relates to N-arylmethylthioanilide compounds useful for the inhibition of the replication of HIV. This invention also relates to a method for the prevention or treatment of HIV-1 infection in a patient which comprises administering to the patient an effective amount of the N-arylmethylthioanilide compounds.

### Background of the Invention

Various compounds have been described as inhibitors of human immunodeficiency virus type 1 (HIV-1) in vitro and are targeted at the virus-encoded reverse transcriptase (RT), e.g., nevirapine, pyridinone, TIBO, BHAP, TSAO, and quinoxaline. U.S. Patents Nos. 5,268,389 and 5,693,827 describe certain compounds useful for inhibiting the replication of HIV. The selectivity of these compounds for HIV-1 is due to a highly specific interaction with HIV-1 RT.

The rapid emergence of HIV-1 strains resistant to several HIV-1 specific RT inhibitors in cell culture and in AIDS patients has caused concern for further development of these inhibitors in the clinic. For example, HIV-1 strains containing the 100 Leu→Ile mutation in their RT are resistant to TIBO R82913 and R82150. HIV-1 strains containing the 138 Glu→Lys mutation in their RT are resistant to TSAO derivatives. The 181 Tyr→Cys mutation in the RT of HIV-1 strains renders the mutant viruses resistant to nevirapine and pyridinone. See, e.g. Balzarini et al, J. Virology 67(9): 5353-5359 (1993) ("Balzarini I") and Balzarini et al. Virology 192: 246-253 (1993) ("Balzarini II"). Attempts have been made to combine various HIV-1 RT inhibitors to eliminate virus resistance. See, e.g., Balzarini I.

U. S. Patent No. 5,696,151 describes certain methylfuranyl- and methylthienyl-pentenylether derivatives useful against HIV-1 and HIV-1 reverse transcriptase mutants.

WO 97 19940 A discloses certain furan- and thiophene carbothioamide derivatives useful as inhibitors of the replication of HIV-1 and HIV-1 mutants.

It is the purpose of this invention to provide new compounds which by themselves, can inhibit or suppress the emergence of wild-type HIV-1 and HIV-1 RT mutant strains. It is also the purpose of this invention to provide a method of preventing or treating HIV-1 infections by administration of such compounds.

### Summary of the Invention

This invention relates to a compound of the formula 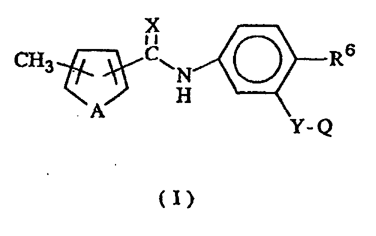 wherein
A and X are independently oxygen or sulphur;
R⁶ is H, halogen, C₁-C₄ alkyl, C₁-C₄ alkoxy, C₁-C₄ alkylthio, cyano, or nitro;
Y is -CH₂O-, -OCH₂-, -CH₂S-, or -CH₂SO₂-;
Q is:
(A) an aromatic group of the structure 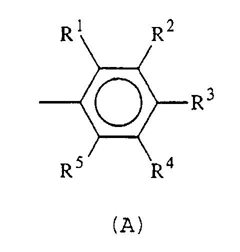 wherein R¹ to R⁵ are each independently:
   (i) hydrogen, halogen, C₁-C₆ alkyl, C₁-C₄ alkoxy, C₁-C₄ alkylthio, C₁-C₄ haloalkoxy, cyano, nitro, hydroxy, acetyloxy, benzoyloxy, amino, acetamido, phenyl, acetyloxymethyl, hydroxymethyl, trihalomethyl, carboxy, (C₁-C₄ alkoxy)carbonyl, formyl , (C₁-C₄ alkyl)carbonyl, benzoyl, or
   (ii) a group of the formula 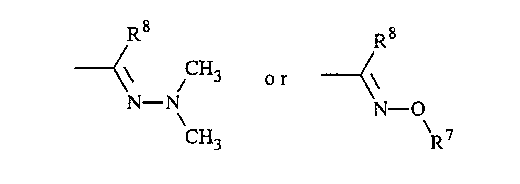
   wherein R⁷ is H, linear or branched C₁-C₄ alkyl, C₁-C₄ haloalkyl, aminocarbonylmethyl, (C₁-C₆ alkoxy)carbonylmethyl, cyanomethyl, or arylmethyl and R⁸ is hydrogen or methyl;
or (B) a group of the formula 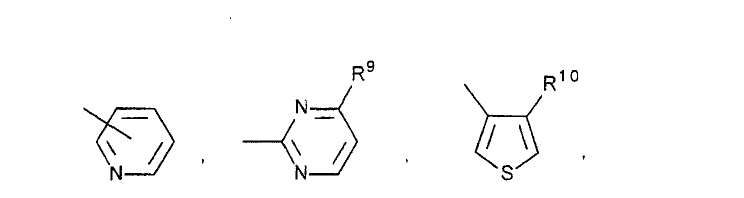 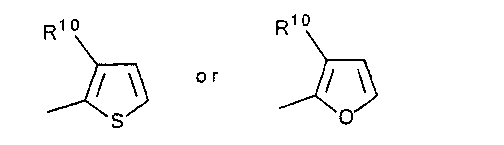 wherein
   R⁹ is hydrogen, C₁-C₄ alkyl, or C₁-C₄ haloalkyl, and
   R¹⁰ is H, halogen, C₁-C₄ alkyl or (C₁-C₄ alkoxy)-carbonyl.

The compounds of this invention are useful for the inhibition of the replication of Human Immunodeficiency Virus-1 (HIV-1), in vitro and in vivo. The compounds are useful in the therapeutic or prophylactic treatment of diseases caused by HIV-1 thereof, such as acquired immune deficiency syndrome (AIDS).

This invention additionally relates to a pharmaceutical composition comprising a therapeutically effective amount of the compound of formula I and a pharmaceutically acceptable carrier.

This invention also relates to a method of treating HIV-1 infection in an afflicted host which comprises administering to the host a therapeutically effective amount of the compound of formula I.

### Description of the Invention

Preferred compounds of this invention are those compounds of formula I wherein:
A is oxygen or sulfur;
X is sulfur;
R⁶ is halogen, C₁-C₄ alkyl, C₁-C₄ alkoxy, C₁-C₆ alkylthio, or cyano
Y is -CH₂O-, -OCH₂-, or -CH₂S-;
Q is an aromatic group of the structure 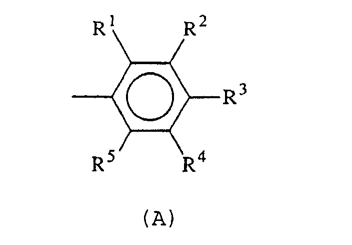
wherein R¹ to R⁵ are each independently hydrogen, halogen, C₁-C₆ alkyl, C₁-C₆ alkoxy, trihalomethyl, cyano, nitro, or trihalomethoxy.

More preferred are those compounds of formula I wherein.
A is oxygen or sulfur;
X is sulfur;
R⁶ is halogen, methoxy, or cyano
Y is -CH₂O- or -OCH₂-;
Q is an aromatic group of the structure 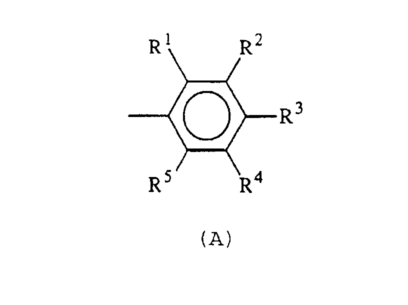 wherein R¹ to R⁵ are each independently hydrogen, fluoro, chloro, methyl, methoxy, trifluoromethyl, trifluoromethoxy, cyano, or nitro, wherein one or more of R², R³, R⁴, and R⁵ are hydrogen.

Particularly preferred is the compound of the formula 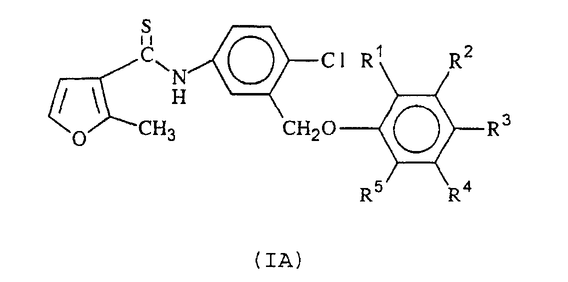 wherein R¹ to R⁵ are each independently hydrogen, fluoro, chloro, methyl, methoxy, trifluoromethyl, trifluoromethoxy, cyano, or nitro, wherein one or more of R², R³, R⁴, and R⁵ are hydrogen.

More particularly preferred are the compounds of formula IA wherein R¹ is fluoro and one or more of R², R³, R⁴, and R⁵ are hydrogen.

### Method of Synthesis

The compounds of this invention can be prepared according to the following scheme (A, X, Y, Q, and R⁶ are as defined above):

### (1) Acid chloride formation

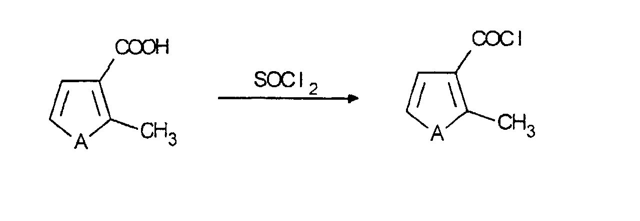

### (2) Substituted protected hydroxymethyl aniline preparation

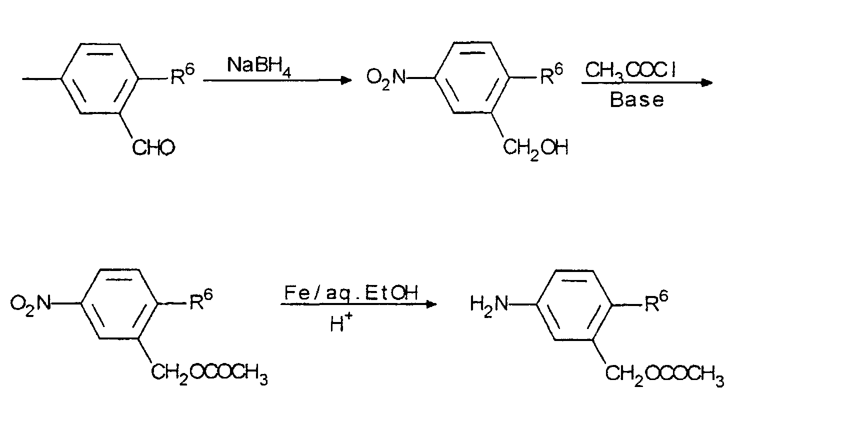

### (3) Amide Formation

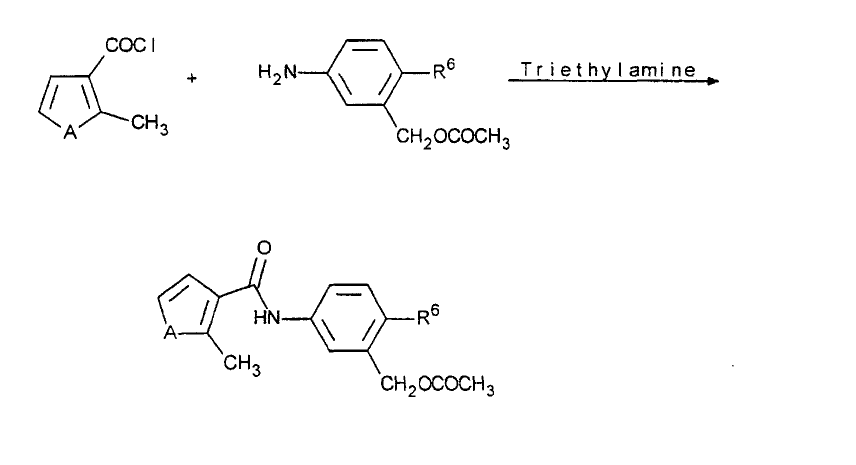

### (4) Deprotection, bromination

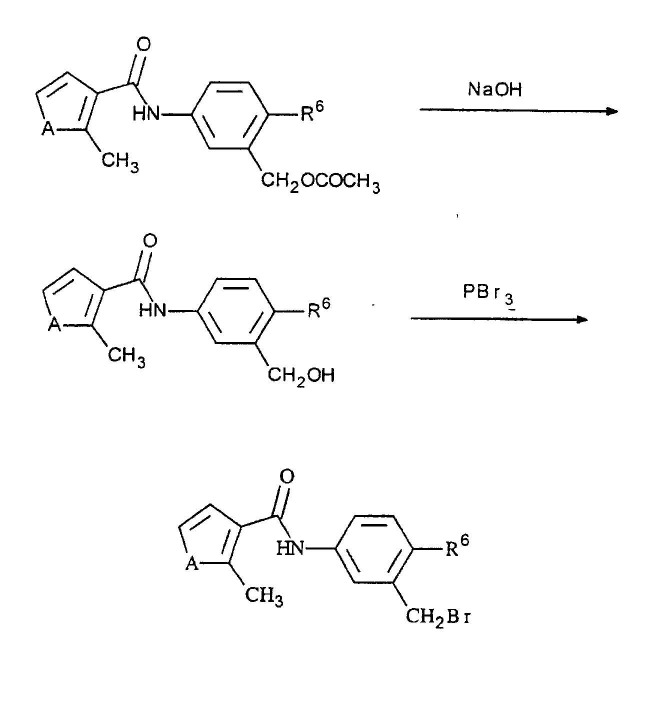

### (5) Arylation

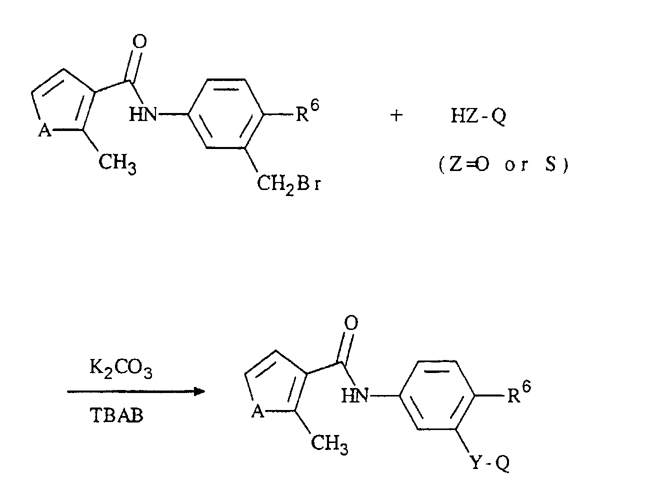

### (6) Thionylation

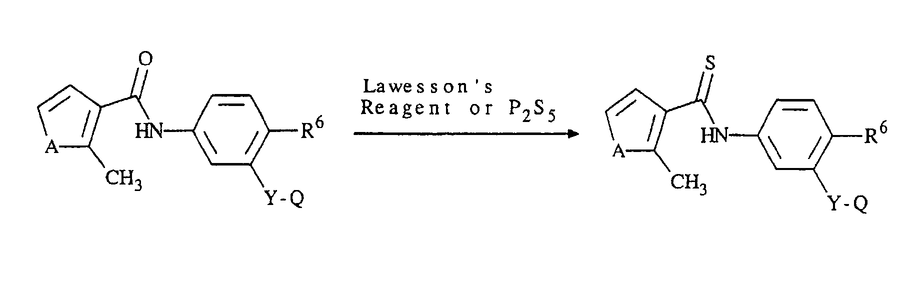

The compounds of the present invention can be administered orally, parenterally, sublingually, by inhalation spray, rectally, or topically in dosage unit formulations containing conventional non-toxic pharmaceutically acceptable carriers, adjuvants, and vehicles. Pharmaceutically acceptable carriers, adjuvants and vehicles useful in the composition of this invention can be found in standard pharmaceutical texts such as, e.g., Remington's Pharmaceutical Sciences, 16th Edition, Mack Publishing Company, Easton, PA (1980).

The therapeutically effective amount of the compounds of this invention that can be combined with the pharmaceutically acceptable carrier to produce a single dosage form will vary depending upon the age and condition of the host treated and the particular mode of administration. In general, the compounds of this invention are most desirably administered at a concentration level that will generally afford anti-virally effective results without causing any harmful or deleterious side effects.

While the compounds of this invention can be administered as the sole active pharmaceutical agents, the compounds can also be used in combination with one or more other pharmaceutical agents which are not deleterious to the activity of the compounds of this invention or whose combination with the compounds will not have a deleterious effect on the host treated.

The following examples are provided to illustrate the present invention.

### EXAMPLES

### Materials and Methods

### Example 1

### Preparation of N-3-((2-chlorophenoxy)methyl)-4-chlorophenyl-2-methyl-3-furancarbothioamide (Compound No. 6)

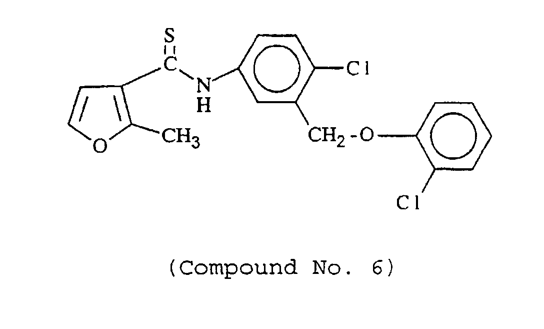

### Step 1: Preparation of 2-chloro-5-nitrobenzoyl alcohol

30g of 2-chloro-5-nitrobenzaldehyde was dissolved in 500 ml of methanol and cooled to 0°C. A solution of 10g of sodium borohydride in 100 ml of water was then added dropwise over 90 minutes while maintaining the temperature below 10°C. The resultant reaction mixture was then stirred for one hour, then acidified with 2N HCl and left stirring overnight. The solids were then, washed with water and dried, to produce 27g of 2-chloro-5-nitrobenzyl alcohol as a white solid.

### Step 2: Preparation of 2-chloro-5-nitrobenzoyl acetate

27g of the 2-chloro-5-nitrobenzyl alcohol prepared above in Step 1, was dissolved in 122ml of toluene. 22ml of triethylamine was then added. The resultant reaction mixture was cooled to 20°C and then a solution of 10.2ml of acetyl chloride in 10ml of toluene, was added dropwise, keeping the temperature below 20°C. The reaction mixture was then stirred overnight. 2.1ml of triethylamine and 1.1ml of acetyl chloride/toluene solution were then added and the reaction mixture was stirred for one hour. 100ml of water was then added, followed by 50ml of ether. The resulting organic phase was separated, washed with 2N HCl, aqueous sodium bicarbonate solution and water. The washed organic phase was then dried over magnesium sulfate and the solvent was evaporated, to produce 29.6g of 2-chloro-5-nitrobenzoyl acetate as a white solid.

### Step 3: Preparation of 5-amino-2-chlorobenzoyl acetate

24g of iron powder was added to a solution of 1.6ml of concentrated HCl, 16.8ml of water, and 70ml of ethanol. 29.6g of the 2-chloro-5-nitrobenzoyl acetate prepared above in Step 2 dissolved in 45ml of ethanol, was then added to the mixture in three equal portions. The resultant reaction mixture was refluxed for 5 hours. An additional 2.4g of iron and 0.1ml of concentrated HCl was then added to the reaction mixture. The reaction mixture was then refluxed for an additional one hour, filtered through Celite and evaporated. 100ml of water was then added to the evaporated material and the resultant mixture was extracted with 100 ml of ether. The ether solution was washed with water, dried over magnesium sulfate, and evaporated, to produce 22.9g of 5-amino-2-chlorobenzoyl acetate as an oil.

### Step 4: Preparation of N-(3-acetoxymethyl-4-chlorophenyl)-2-methyl-3-furancarboxanilide

A solution of 22.8g of the 5-amino-2-chlorobenzoyl acetate from Step 3 above and 17.2ml of triethylamine in 118ml ether was prepared and then added dropwise to a second solution of 16.6g 2-methyl-3-thiophenecarboxylic acid chloride in 118ml ether at 0°C to 10°C and the resultant mixture was stirred at room temperature overnight. 100ml of water and 100ml of ethyl acetate were then added to the mixture, the organic phase separated, washed with 2N hydrochloric acid and water, dried over magnesium sulfate, and the solvents removed in vacuo, to produce 29.87g of N-(3-acetoxymethyl-4-chlorophenyl)-2-methyl-3-furancarboxamide as a beige solid.

### Step 5: Preparation of N-(4-chloro-3-hydroxymethylphenyl)-2-methyl-3-furancarboxamide

A solution of 29g of the N-(3-acetoxymethyl-4-chlorophenyl)-2-methyl-3-furancarboxamide prepared in Step 4 above and 14.5g potassium hydroxide in 110ml water, was prepared. The solution was then heated at 70°C for 16 hours and then acidified with 2N hydrochloric. The solid so produced collected, washed with water, and dried, producing 23.65g of N-(4-chloro-3-hydroxymethylphenyl)-2-methyl-3-furancarboxamide as a white solid.

### Step 6: Preparation of N-(3-bromomethyl-4-chlorophenyl)-2-methyl-3-furancarboxamide

12g of the N-(4-chloro-3-hydroxymethylphenyl)-2-methyl-3-furancarboxamide prepared in Step 5 above, was dissolved in 180ml ethyl acetate. 1.8ml of phosphorus tribromide was then added. The resultant mixture was stirred for 90 minutes at room temperature. 100ml of water was then added to the mixture. The resultant organic phase was separated, washed with water, aqueous sodium bicarbonate solution and water, and then dried over magnesium sulfate. The solvent was evaporated off to produce 12.97g of N-(3-bromomethyl-4-chlorophenyl)-2-methyl-3-furancarboxamide as a solid.

### Step 7: Preparation of N-3-((2-chlorophenoxy)methyl)-4-chlorophenyl-2-methyl-3-furancarboxamide

2g of the N-(3-bromomethyl-4-chlorophenyl)-2-methyl-3-furancarboxamide produced in Step 6, was dissolved in 20ml of 2-butanone to produce a solution. 0.84g of potassium carbonate, 0.79g of 2-chlorophenol and 0.2g of tetrabutylammonium bromide were then added to the solution. The resultant reaction mixture was stirred at room temperature overnight, the solvents removed in vacuo, and the residue extracted with ethyl acetate, to produce a second solution. This second solution was washed with 2N aqueous sodium hydroxide and water, and then dried over magnesium sulfate. The solvent was removed to produce 2.7g of a solid, which was purified by dissolving in ethyl acetate:hexane (20:80) and running the resultant solution through a plug of silica gel. Removal of solvent produced 2.0g of N-3-((2-chlorophenoxy)methyl)-4-chlorophenyl-2-methyl-3-furancarboxamide as a white solid.

### Step 8 Preparation of N-3-((2-chlorophenoxy)methyl)-4-chlorophenyl-2-methyl-3-furancarbothioamide

1.5g of the N-3-((2-chlorophenoxy)methyl)-4-chlorophenyl-2-methyl-3-furancarboxamide prepared in Step 7 above, 0.8g of Lawesson's reagent (0.8 g) and 1.6g of sodium bicarbonate were added to 35ml of toluene, and the resultant reaction mixture was refluxed for five hours. The reaction mixture was then passed through a plug of neutral aluminum oxide, eluted with 1:1 ether/hexane and purified by column chromatography on silica gel, to produce 0.77g of N-3-((2-chlorophenoxy)methyl)-4-chlorophenyl-2-methyl-3-furancarbothioamide as a yellow solid, mp 116-117°C. Nuclear magnetic resonance and mass spectra were consistent with the claimed structure.

The other compounds listed in Table 1 were prepared in similar manner.

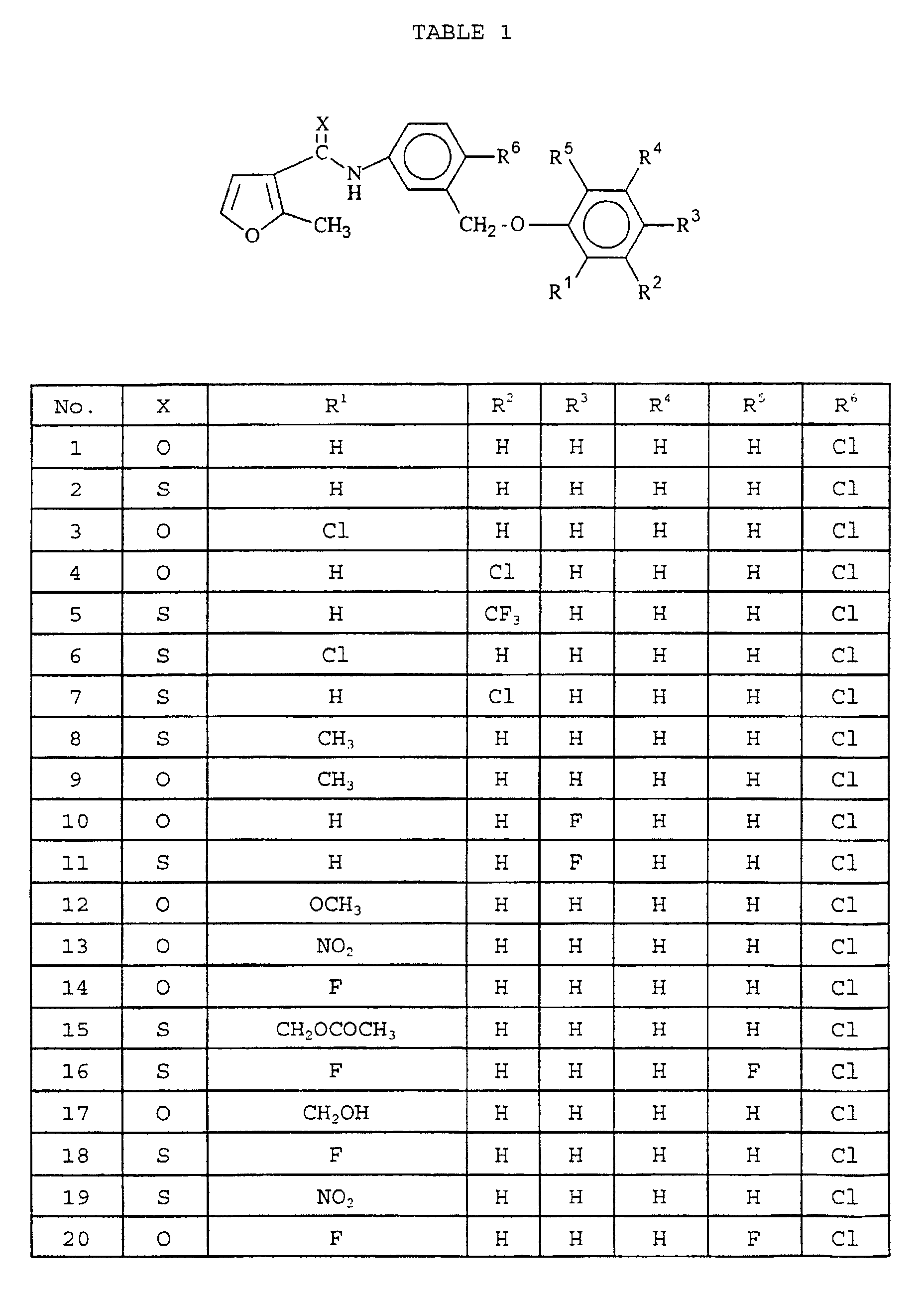

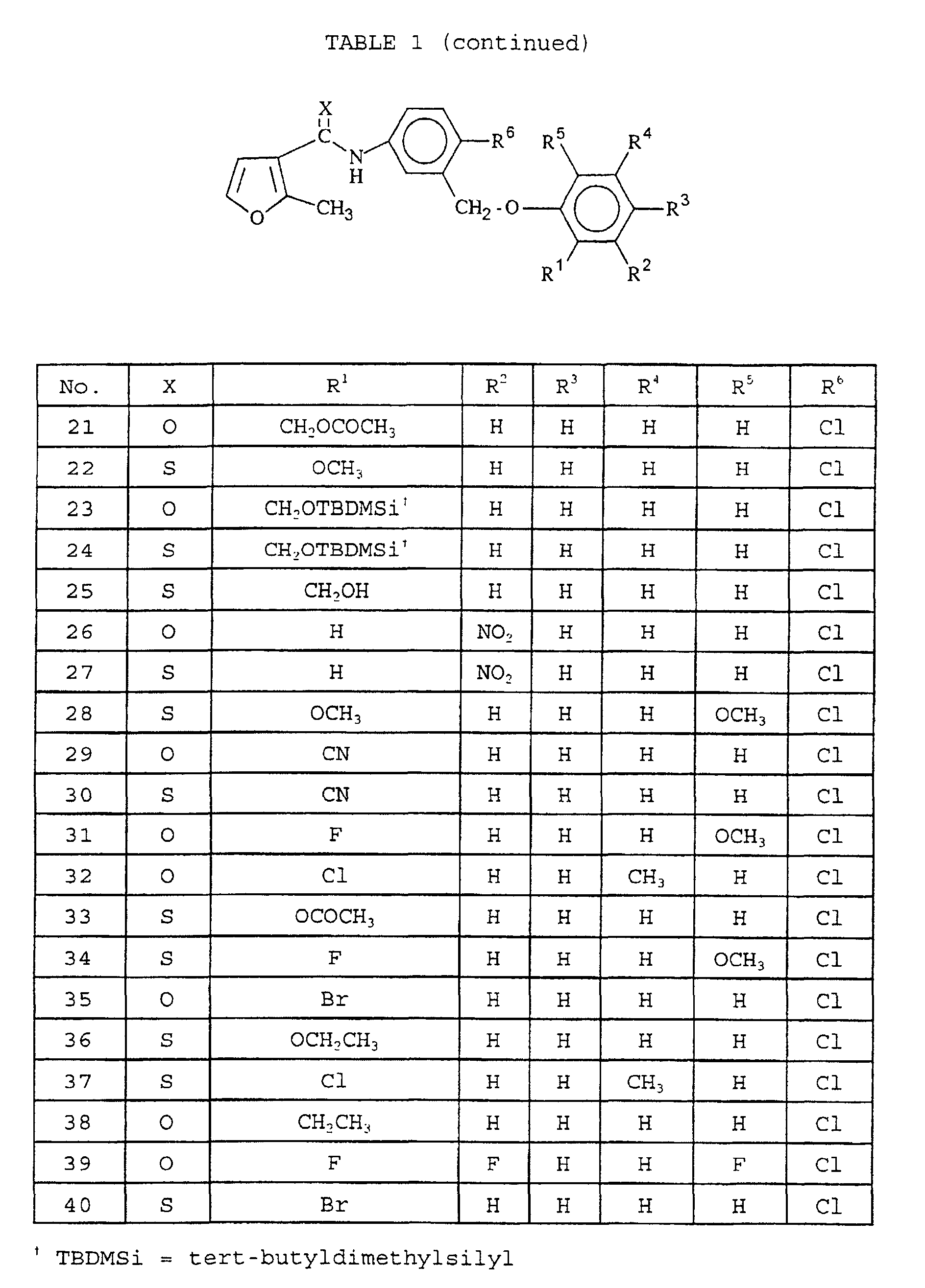

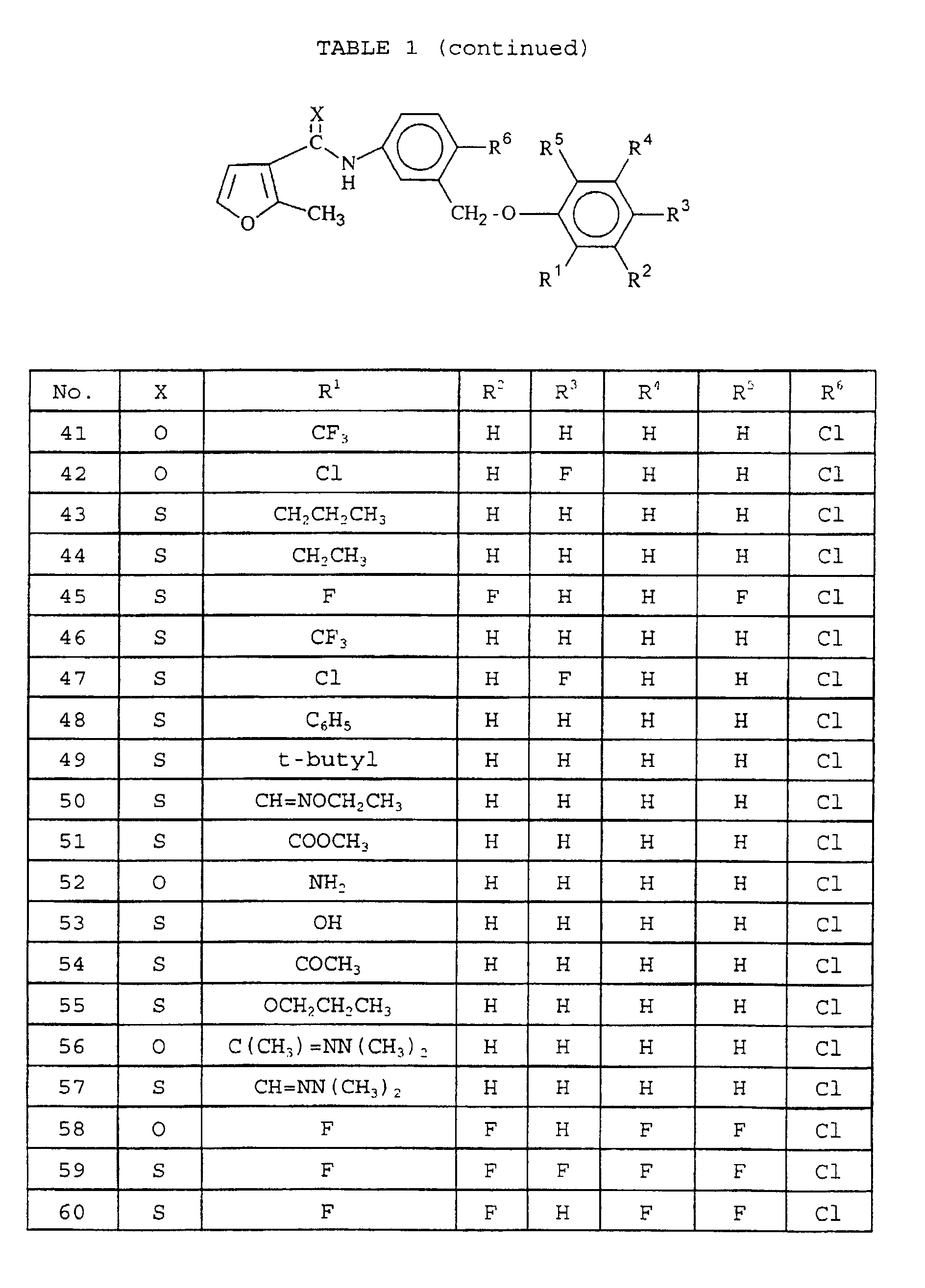

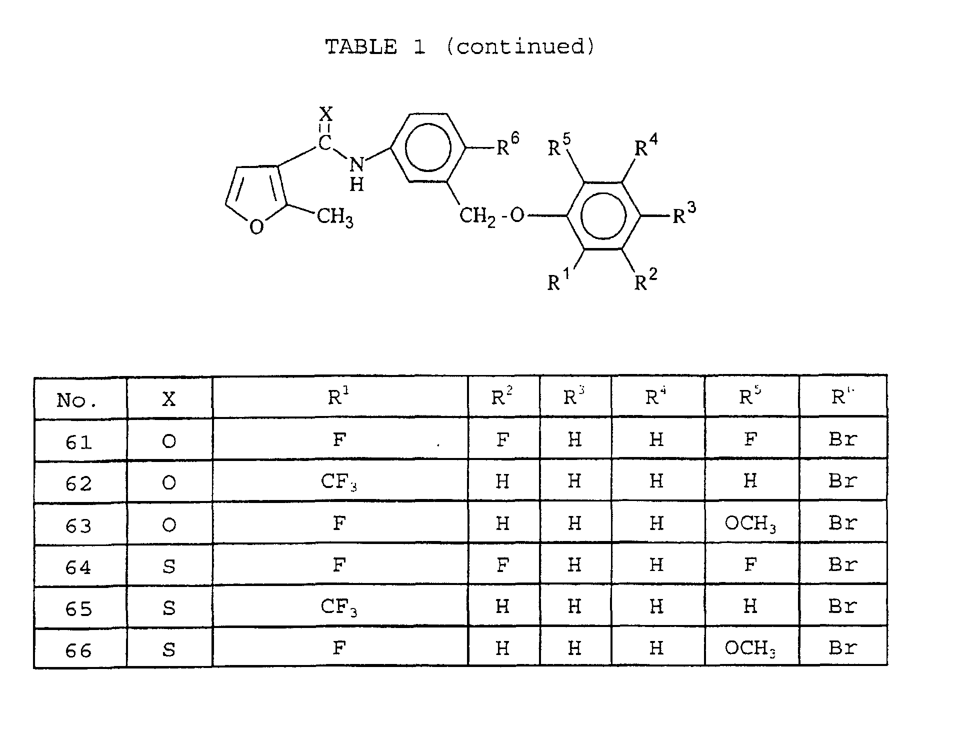

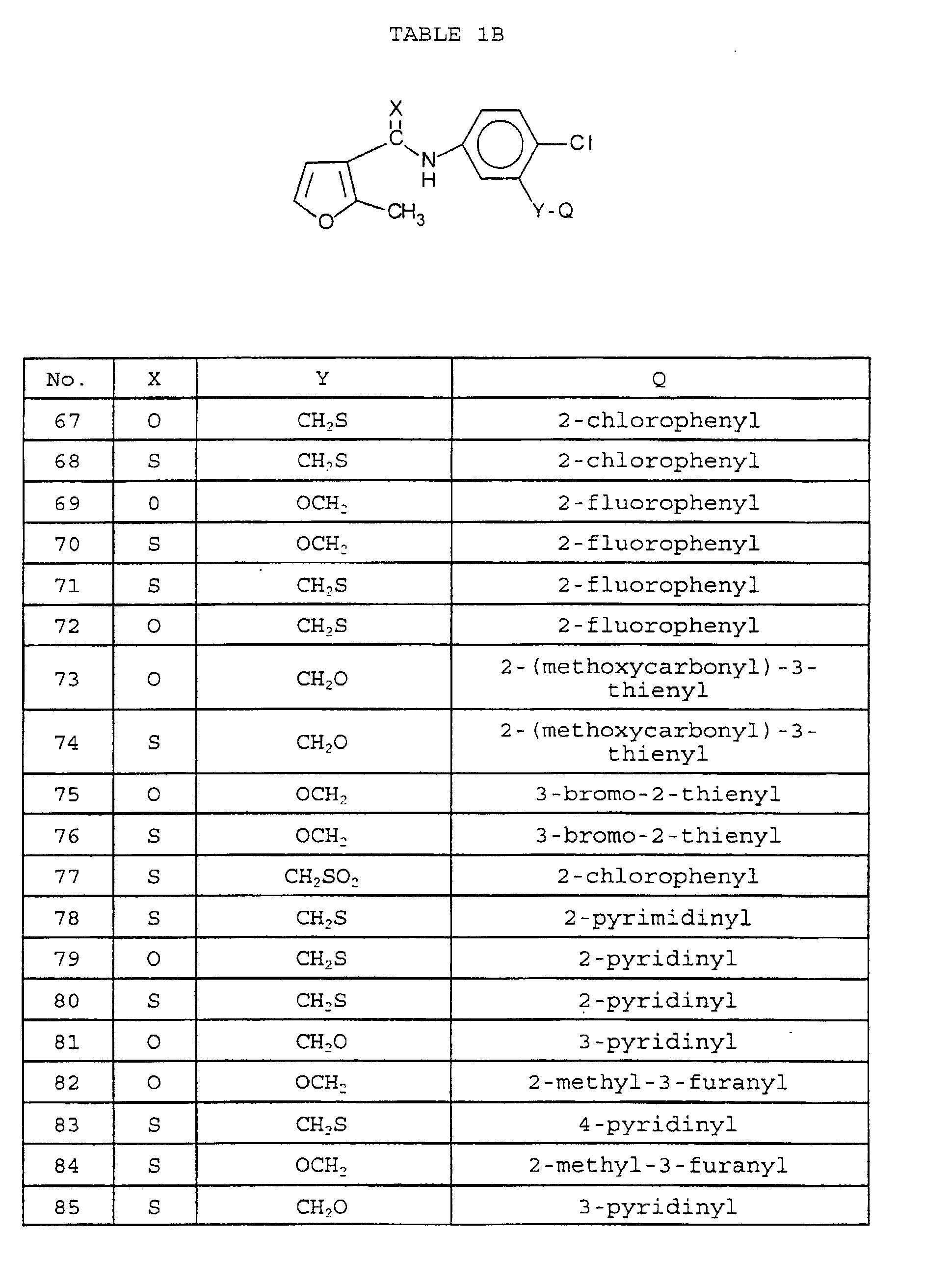

### Cells and Viruses

CEM cells were obtained from the American Tissue Cell Culture Collection (Rockville, Md.). HIV-1(III_{B}) was originally obtained from the culture supernatant of persistently HIV-1-infected H9 cells and was provided by R.C. Gallo and M. Popovic (National Cancer Institute, National Institutes of Health, Bethesda, MD).

The selection and characterization of the HIV-1 RT mutant strains were done as follows: HIV-1/100-Ile ("100-Ile") was selected for resistance against TIBO R82150 as described in Balzarini et al, Virology 192: 246-253 (1993); HIV-1/103-Asn ("103-Asn") was selected for resistance against TIBO R82913 as described in Balzarini et al, Virology 192: 246-253 (1993); HIV-1/106-Ala ("106-Ala") was selected for resistance against nevirapine as described in Balzarini et al, J. Virol. 67: 5353-5359 (1993); HIV-1/Lys-138 ("Lys-138") was selected for resistance against TSAO-m³T as described in Balzarini et al, Virology 192: 246-253 (1993) and Balzarini et al, Proc. Nat. Acad. Sci. USA 90: 6952-6956 (1993); HIV-1/181-Cys ("181-Cys") was selected for resistance against pyridinone L-697,661 as described in Balzarini et al, Virology 192: 246-253 (1993); and HIV-1/188-His ("188-His") was selected for resistance against HEPT as described in Balzarini et al, Mol. Pharmocol. 44: 694-701 (1993). 188-His was then further converted to HIV-1/188-Leu("188-Leu") upon further passage in cell culture in the absence of the HEPT. HIV-1/101-Glu ("101-Glu") and HIV-1/190-Glu ("190-Glu") were selected for resistance against the thiocarboxanilide derivative designated as UC38 as described in Balzarini et al, Antiviral Research 27: 219-236 (1995). HIV-1/184-Ile ("184-Ile") was selected for resistance against the combination of 3TC and TSAO-m³T as described in Balzarini et al, Molecular Pharm. 49: 882-890 (1996). HIV-1/184-Val ("184-Val") was selected for resistance against 3TC as described in Balzarini et al, Molecular Pharm. 49: 882-890 (1996).

### Antiviral activity of the test compounds in cell cultures

CEM cells were suspended at ≈300,000 cells per ml of culture medium and infected with approximately 100 CCID₅₀ (CCID₅₀ being the 50% cell culture infective dose) of HIV-1(III_{B}) (designated as "WT" in Table 3) or one of the HIV-1 RT mutant strains described above. Then 100 µl of the infected cell suspensions was added to 200 µl microtiter plate wells containing 100 µl of appropriate serial (5-fold) dilutions of the test compounds. The inhibitiory effect of the test compounds on HIV-1 induced syncytium formation in CEM cells was examined microscopically on day 4 post infection. The 50% effective concentration (EC₅₀) was defined as the test compound concentration that inhibits syncytium formation in the HIV-1-infected cell cultures by 50%.

**TABLE 2**

| **Activity Against HIV-1(III**_{**B**}**)** | |
|---|---|
| Compound No. | EC₅₀ (mmol/ml) |
| 1 | 2.38×10⁻⁶, 1.55×10⁻⁶, 3.20×10⁻⁶, 4.81×10⁻⁶ |
| 2 | 2.28×10⁻⁷, 1.61×10⁻⁷, 9.86×10⁻⁸, 6.76×10⁻⁸ |
| 5 | 3.83×10⁻⁸, 3.88×10⁻⁶, 4.11×10⁻⁶, 3.84×10⁻⁶ |
| 6 | 5.86×10⁻⁸, 1.04×10⁻⁷ |
| 7 | 6.82×10⁻⁸, 5.25×10⁻⁸, 4.32×10⁻⁷ |
| 8 | 1.07×10⁻⁷ |
| 9 | 1.35×10⁻⁵, 3.50×10⁻⁵ |
| 10 | 4.15×10⁻⁶, 4.26×10⁻⁶, 3.79×10⁻⁶ |
| 11 | 8.51×10⁻⁸, 1.61×10⁻⁷, 3.65×10⁻⁷, 2.76×10⁻⁷ |
| 12 | 4.88×10⁻⁷, 3.79×10⁻⁷, 4.42×10⁻⁷, 8.99×10⁻⁷ |
| 13 | 3.01×10⁻⁷, 2.08×10⁻⁷, 1.51×10⁻⁷, 3.58×10⁻⁷ |
| 14 | 7.71×10⁻⁷, 3.95×10⁻⁷, 6.64×10⁻⁷, 2.55×10⁻⁷ |
| 16 | 2.86×10⁻⁸, 3.92×10⁻⁸ |
| 17 | 1.65×10⁻⁵, 4.81×10⁻⁶ |
| 18 | 1.17×10⁻⁷, 1.11×10⁻⁷ |
| 19 | 2.71×10⁻⁸, 3.93×10⁻⁸ |
| 20 | 4.96×10⁻⁷, 7.99×10⁻⁷ |
| 22 | 1.60×10⁻⁷, 2.09×10⁻⁷ |
| 25 | 3.14×10⁻⁶, 1.09×10⁻⁶, 2.72×10⁻⁶, 1.49×10⁻⁶, 1.15×10⁻⁶, 1.00×10⁻⁶ |
| 26 | 4.61×10⁻⁶, 4.14×10⁻⁶, 4.94×10⁻⁶, 3.66×10⁻⁶ |
| 27 | 1.21×10⁻⁷, 3.40×10⁻⁷, 5.95×10⁻⁷ |
| 28 | 6.71×10⁻⁶, 7.56×10⁻⁶, 9.05×10⁻⁸, 1.96×10⁻⁵, 1.01×10⁻⁵, 1.10×10⁻⁵ |
| 29 | 3.39×10⁻⁶, 3.48×10⁻⁶, 3.33×10⁻⁶, 3.40×10⁻⁶, 3.10×10⁻⁶, 3.76×10⁻⁶ |
| 30 | 6.35×10⁻⁸, 6.87×10⁻⁸, 9.79×10⁻⁸, 1.03×10⁻⁷ |
| 31 | 7.78×10⁻⁸, 1.24×10⁻⁷, 1.97×10⁻⁷, 2.13×10⁻⁷ |
| 32 | 4.61×10⁻⁶, 2.74×10⁻⁵, 9.85×10⁻⁶, 1.12×10⁻⁵, 2.57×10⁻⁶ |
| 33 | 1.39×10⁻⁶, 2.83×10⁻⁶, 4.16×10⁻⁶, 2.67×10⁻⁶ |
| 34 | 5.65×10⁻⁹, 1.74×10⁻⁸ |
| 35 | 2.29×10⁻⁶, 3.14×10⁻⁶, 1.99×10⁻⁶, 1.23×10⁻⁶ |
| 36 | 3.77×10⁻⁷, 1.24×10⁻⁷, 1.21×10⁻⁶, 7.88×10⁻⁷ |
| 37 | 1.92×10⁻⁸, 7.66×10⁻⁸ |
| 38 | 2.88×10⁻⁶, 1.45×10⁻⁶, 4.70×10⁻⁶ |
| 39 | 1.02×10⁻⁷, 5.07×10⁻⁸, 1.02×10⁻⁷, 1.13×10⁻⁷ |
| 40 | 3.53×10⁻⁸, 4.60×10⁻⁸, 7.11×10⁻⁸, 1.68×10⁻⁸ |
| 41 | 7.61×10⁻⁷, 1.11×10⁻⁶, 1.56×10⁻⁶ |
| 42 | 4.66×10⁻⁶, 1.82×10⁻⁵ |
| 43 | 4.25×10⁻⁸, 1.18×10⁻⁷ |
| 44 | 3.07×10⁻⁸, 1.80×10⁻⁸, 6.23×10⁻⁸, 9.54×10⁻⁸ |
| 45 | 6.35×10⁻⁸, 6.50×10⁻¹⁰, 1.46×10⁻⁹, 7.29×10⁻⁹, 2.69×10⁻⁹ |
| 46 | 3.93×10⁻⁸, 5.48×10⁻⁸, 2.11×10⁻⁸, 9.14×10⁻⁹ |
| 47 | 1.03×10⁻⁶, 5.16×10⁻⁷ |
| 48 | 3.11×10⁻⁶, 2.75×10⁻⁶, 6.11×10⁻⁶, 5.41×10⁻⁶ |
| 49 | 3.84×10⁻⁷, 5.99×10⁻⁷, 4.01×10⁻⁷, 1.13×10⁻⁶ |
| 50 | 1.43×10⁻⁶, 1.43×10⁻⁷, 9.06×10⁻⁷, 1.07×10⁻⁶ |
| 51 | 1×10⁻⁶, 2.26×10⁻⁷, 9.15×10⁻⁷, 5.68×10⁻⁷ |
| 52 | 1.37×10⁻⁵, 1.62×10⁻⁵, 3.28×10⁻⁵ |
| 53 | 4.34×10⁻⁶, 3.93×10⁻⁸, 2.79×10⁻⁶, 4.58×10⁻⁶ |
| 54 | 6.67×10⁻⁶, 1.53×10⁻⁵, 5.64×10⁻⁶ |
| 55 | 1.0×10⁻⁵, 9.0×10⁻⁶, 3.73×10⁻⁶, 4.26×10⁻⁶ |
| 56 | 2.23×10⁻⁶, 2.91×10⁻⁶, 3.54×10⁻⁶, 3.58×10⁻⁶ |
| 57 | 2.14×10⁻⁷, 7.66×10⁻⁸, 1.21×10⁻⁷, 7.92×10⁻⁸ |
| 58 | 4.75×10⁻⁷, 2.81×10⁻⁷ |
| 59 | 1.99×10⁻⁷, 1.17×10⁻⁷ |
| 60 | 1.06×10⁻⁸, 4.62×10⁻⁹ |
| 61 | 2.05×10⁻⁹ |
| 62 | 1.95×10⁻⁷, 8.17×10⁻⁷, 5.09×10⁻⁷, 5.52×10⁻⁷, 7.93×10⁻⁷, 5.54×10⁻⁷ |
| 63 | 7.45×10⁻⁹, 7.55×10⁻⁹ |
| 64 | 6.21×10⁻¹¹, 5.87×10⁻⁹ |
| 65 | 2.90×10⁻¹⁰, 2.81×10⁻¹⁰ |
| 69 | 3.59×10⁻⁷, 3.17×10⁻⁷, 1.26×10⁻⁷, 2.44×10⁻⁷ |
| 70 | 1.22×10⁻⁸, 9.67×10⁻⁹ |
| 71 | 3.42×10⁻⁷, 1.28×10⁻⁷, 1.60×10⁻⁷, 3.96×10⁻⁷ |
| 72 | 1.49×10⁻⁵, 1.21×10⁻⁵, 5.80×10⁻⁶ |
| 73 | 1.31×10⁻⁴, 2.12×10⁻⁵ |
| 74 | 2.70×10⁻⁷, 1.31×10⁻⁷, 9.28×10⁻⁷, 1.47×10⁻⁷ |
| 75 | 5.88×10⁻⁷, 1.50×10⁻⁶, 1.09×10⁻⁶, 2.60×10⁻⁶ |
| 76 | 8.61×10⁻⁸, 9.52×10⁻⁸ |
| 77 | 2.51×10⁻⁷, 2.99×10⁻⁷, 4.05×10⁻⁷, 1.11×10⁻⁶ |
| 78 | 3.02×10⁻⁷, 6.96×10⁻⁷, 7.12×10⁻⁷, 3.75×10⁻⁷ |
| 79 | 2.83×10⁻⁶, 2.99×10⁻⁶, 4.04×10⁻⁶, 3.30×10⁻⁶, 1.65×10⁻⁶, 6.14×10⁻⁷ |
| 80 | 2.18×10⁻⁷, 1.50×10⁻⁷ |
| 81 | 5.50×10⁻⁶, 3.69×10⁻⁶, 1.49×10⁻⁵ |
| 82 | 1.46×10⁻⁶, 3.15×10⁻⁶, 1.25×10⁻⁶, 2.43×10⁻⁶ |
| 83 | 1.65×10⁻⁵, 1.33×10⁻⁵, 1.05×10⁻⁵, 1.21×10⁻⁵ |
| 84 | 6.61×10⁻⁸, 2.55×10⁻⁷, 1.26×10⁻⁷ |
| 85 | 1.51×10⁻⁷, 2.11×10⁻⁷ |

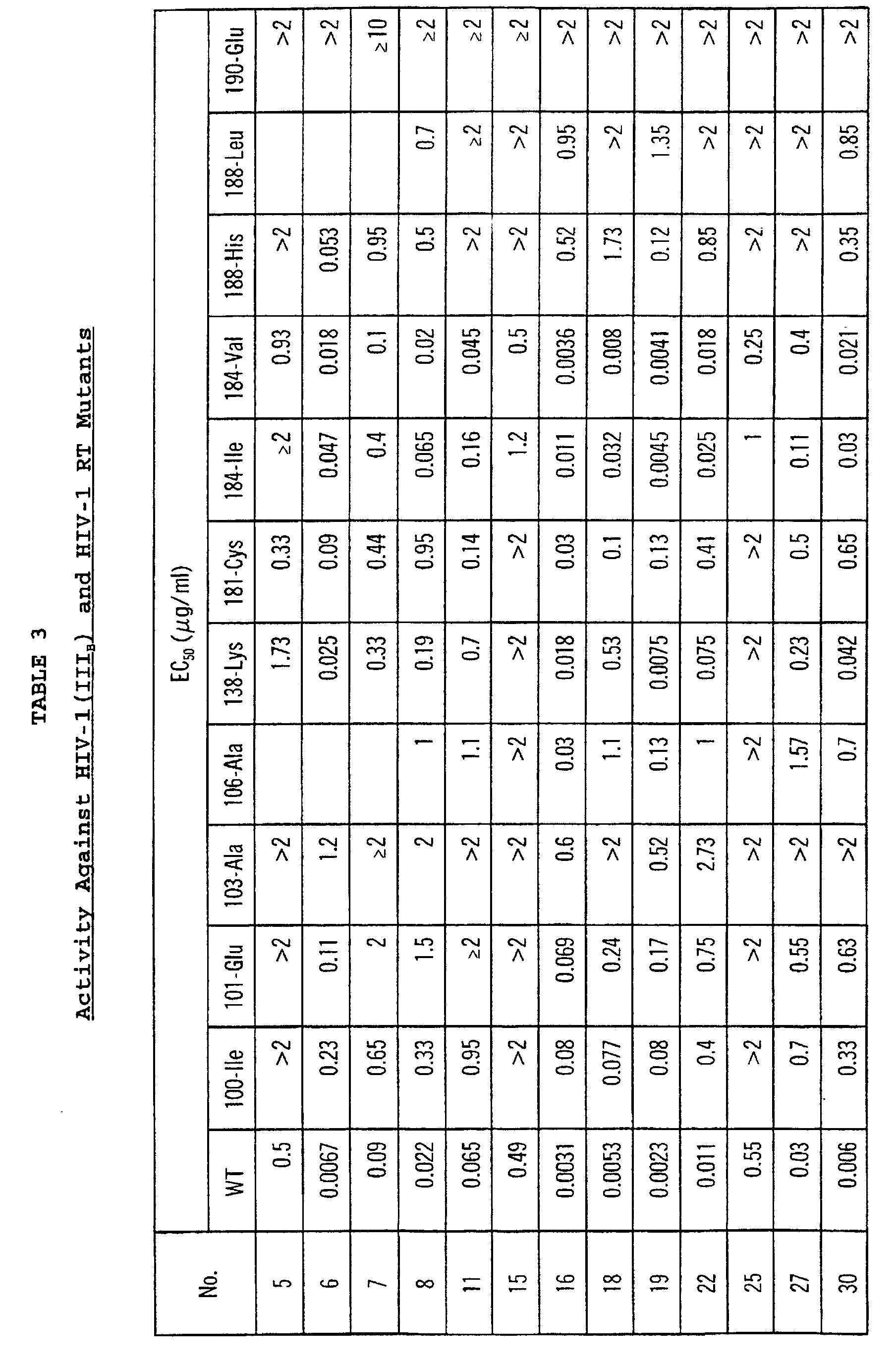

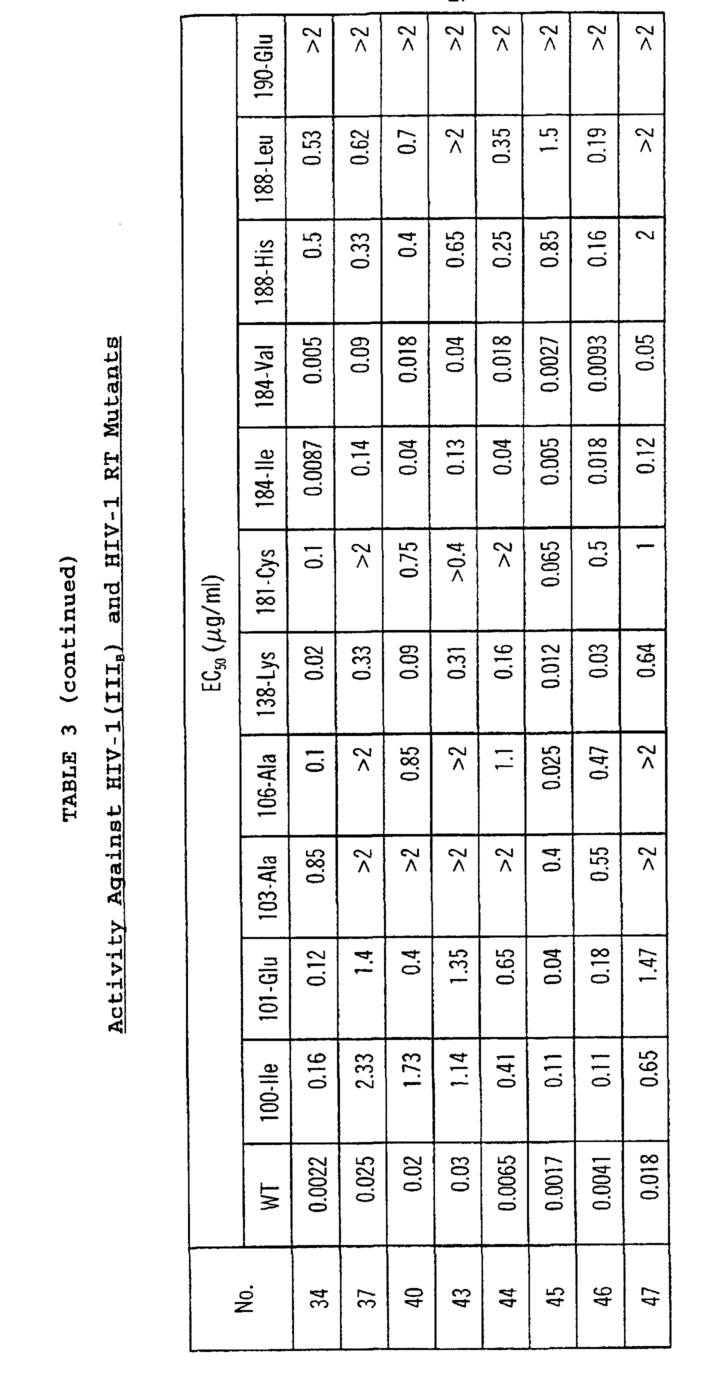

## Claims

1. A compound of the formula 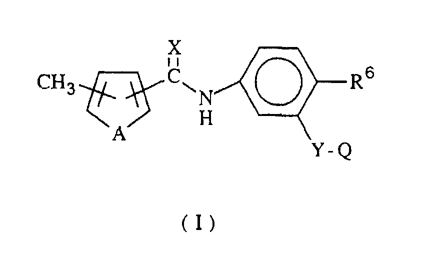 wherein
A and X are independently oxygen or sulfur;
R⁶ is H, halogen, C₁-C₄ alkyl, C₁-C₄ alkoxy, C₁-C₄ alkylthio, cyano, or nitro;
Y is -CH₂O-, -OCH₂-, -CH₂S-, or -CH₂SO₂-;
Q is:
(A) a group of the structure 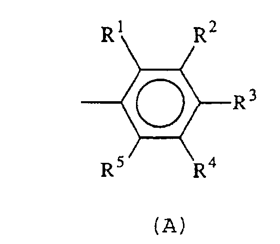 wherein R¹ to R⁵ are each independently:
(i) hydrogen, halogen,C₁-C₆ alkyl, C₁-C₄ alkoxy, C₁-C₄ alkylthio, C₁-C₄ haloalkoxy, cyano, nitro, hydroxy, acetyloxy, benzoyloxy, amino, acetamido, phenyl, acetyloxymethyl, hydroxymethyl, trihalomethyl, carboxy, (C₁-C₄ alkoxy)carbonyl, formyl, (C₁-C₄ alkyl)carbonyl, benzoyl, or
(ii) a group of the formula 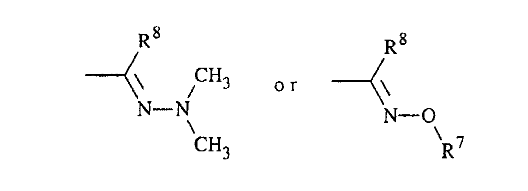
wherein R⁷ is H, linear or branched C₁-C₄ alkyl, C₁-C₄ haloalkyl, aminocarbonylmethyl, (C₁-C₆ alkoxy)carbonylmethyl, cyanomethyl, or arylmethyl and R⁸ is hydrogen or methyl;
or (B) a group of the formula 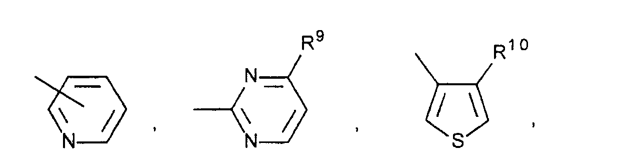 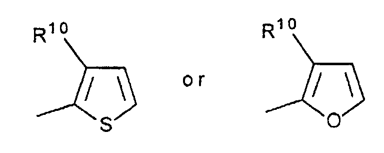 wherein
R⁹ is hydrogen, C₁-C₄ alkyl, or C₁-C₄ haloalkyl, and
R¹⁰ is H, halogen, C₁-C₄ alkyl or (C₁-C₄ alkoxy)-carbonyl.

2. A compound as recited in claim 1 wherein:
A is oxygen or sulfur;
X is sulfur;
R⁶ is halogen, C₁-C₄ alkyl, C₁-C₄ alkoxy, C₁-C₆ alkylthio, or cyano
Y is -CH₂O-, -OCH₂-, or -CH₂S-;
Q is a group of the structure 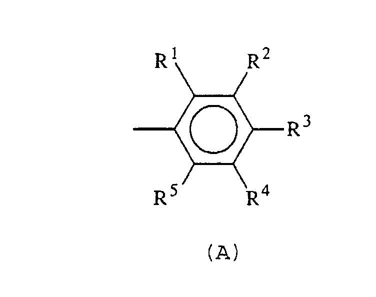
wherein R¹ to R⁵ are each independently hydrogen, halogen, C₁-C₆ alkyl, C₁-C₆ alkoxy, trihalomethyl, cyano, nitro, or trihalomethoxy.

3. A compound as recited in claim 2 wherein
R⁶ is halogen, methoxy, or cyano
Y is -CH₂O- or -OCH₂-;
Q is an aromatic group of the structure 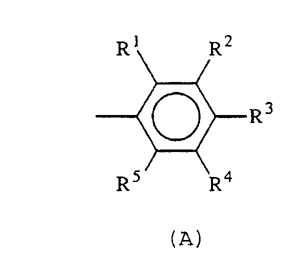 wherein R¹ to R⁵ are each independently hydrogen, fluoro, chloro, methyl, methoxy, trifluoromethyl, trifluoromethoxy, cyano, or nitro, wherein one or more of R², R³, R⁴, and R⁵ are hydrogen.

4. A compound of the formula 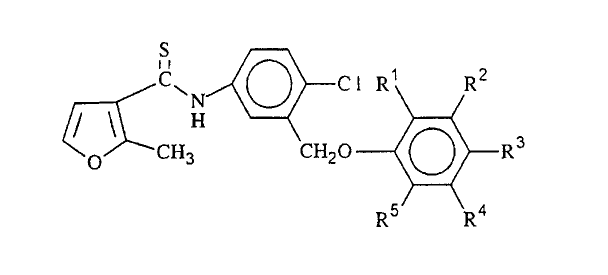 wherein R¹ to R⁵ are each independently hydrogen, fluoro, chloro, methyl, methoxy, trifluoromethyl, trifluoromethoxy, cyano, or nitro, wherein one or more of R², R³, R⁴, and R⁵ are hydrogen.

5. A compound as recited in claim 4 wherein R¹ and R⁵ are fluorine, and R², R³, and R⁴ are hydrogen.

6. A compound as recited in claim 4 wherein R¹ is nitro, and R², R³, R⁴, and R⁵ are hydrogen.

7. A compound as recited in claim 4 wherein R¹ is fluoro, R⁵ is methoxy, and R², R³, and R⁴ are hydrogen.

8. A compound as recited in claim 4 wherein R¹ is bromo, and R², R³, R⁴ and R⁵ are hydrogen.

9. A compound as recited in claim 4 wherein R¹, R² and R⁵ are fluoro, and R³ and R⁴ are hydrogen.

10. A compound as recited in claim 4 wherein R¹ is trifluoromethyl, and R², R³, R⁴ and R⁵ are hydrogen.

11. A compound as recited in claim 4 wherein R¹, R², R³ and R⁵ are fluoro, and R⁴ is hydrogen.

12. A compound as recited in claim 4 wherein R¹ is fluoro, and R², R³, R⁴, and R⁵ are hydrogen.

13. A compound of the formula

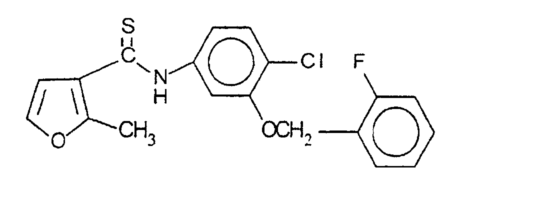

14. A pharmaceutical composition useful for treating HIV-1 infection in an infected host, which composition comprises a therapeutically effective amount of the compound as recited in claim 1 and a pharmaceutically acceptable carrier.

15. A pharmaceutical composition useful for treating HIV-1 infection in an infected host, which composition comprises a therapeutically effective amount of the compound as recited in claim 2 and a pharmaceutically acceptable carrier.

16. A pharmaceutical composition useful for treating HIV-1 infection in an infected host, which composition comprises a therapeutically effective amount of the compound as recited in claim 3 and a pharmaceutically acceptable carrier.

17. A pharmaceutical composition useful for treating HIV-1 infection in an infected host, which composition comprises a therapeutically effective amount of the compound as recited in claim 4 and a pharmaceutically acceptable carrier.

18. A pharmaceutical composition useful for treating HIV-1 infection in an infected host, which composition comprises a therapeutically effective amount of the compound as recited in claim 13 and a pharmaceutically acceptable carrier.

19. Use of the compound as recited in one of the claims 1 to 4 and 13 for the manufacture of a medicament for treating HIV-1 infection in an afflicted host.

20. Use of a compound as recited in one of claims 1 to 4 and 13 for the manufacture of a medicament for inhibiting the replication of HIV-1.

## Patentansprüche

1. Verbindung der Formel 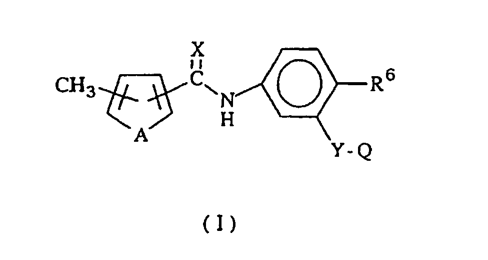 worin
A und X unabhängig voneinander für Sauerstoff oder Schwefel stehen,
R⁶ für H, Halogen, C₁-C₄ Alkyl, C₁-C₄ Alkoxy, C₁-C₄ Alkylthio, Cyano oder Nitro steht,
Y für -CH₂O-, -OCH₂-, -CH₂S- oder -CH₂SO₂- steht,
Q für
(A) eine aromatische Gruppe der Struktur 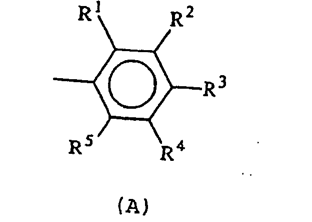 worin R¹ bis R⁵ jeweils unabhängig voneinander stehen für
(i) Wasserstoff, Halogen, C₁-C₆ Alkyl, C₁-C₄ Alkoxy, C₁-C₄ Alkylthio, C₁-C₄ Halogenalkoxy, Cyano, Nitro, Hydroxy, Acetyloxy, Benzoyloxy, Amino, Acetamido, Phenyl, Acetyloxymethyl, Hydroxymethyl, Trihalogenmethyl, Carboxy, (C₁-C₄ Alkoxy)carbonyl, Fonnyl, (C₁-C₄ Alkyl)carbonyl, Benzoyl oder
(ii) eine Gruppe der folgenden Fonnel 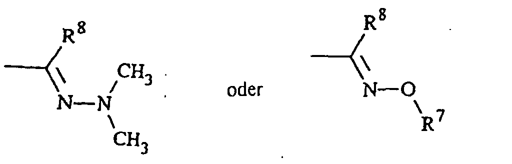
worin R⁷ für H, lineares oder verzweigtes C₁-C₄ Alkyl, C₁-C₄ Halogenalkyl, Aminocarbonylmethyl, (C₁-C₆ Alkoxy)carbonylmethyl, Cyanomethyl oder Arylmethyl steht und R⁸ Wasserstoff oder Methyl bedeutet,
oder
(B) eine Gruppe der Formel 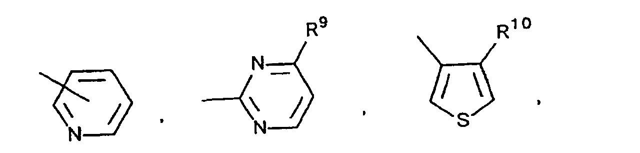 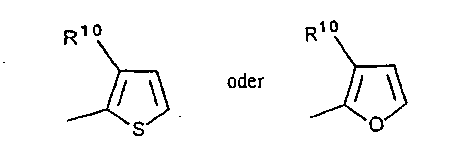
worin R⁹ für Wasserstoff, C₁-C₄ Alkyl oder C₁-C₄ Halogenalkyl steht und
R¹⁰ für H, Halogen, C₁-C₄ Alkyl oder (C₂-C₄ Alkoxy)carbonyl steht.

2. Verbindung nach Anspruch 1, worin
A für Sauerstoff oder Schwefel steht,
X für Schwefel steht,
R⁶ für Halogen, C₁-C₄ Alkyl, C₁-C₄ Alkoxy, C₁-C₆ Alkylthio oder Cyano steht,
Y für -CH₂O-, -OCH₂- oder -CH₂S- steht,
Q für eine Gruppe der folgenden Struktur steht 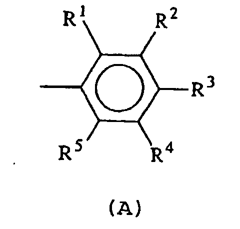 worin R¹ bis R⁵ jeweils unabhängig voneinander stehen für Wasserstoff, Halogen, C₁-C₆ Alkyl, C₁-C₆ Alkoxy, Trihalogenmethyl, Cyano, Nitro oder Trihalogenmethoxy.

3. Verbindung nach Anspruch 2, worin
R⁶ für Halogen, Methoxy oder Cyano steht,
Y für -CH₂O- oder -OCH₂- steht,
Q für eine aromatische Gruppe der folgenden Struktur steht 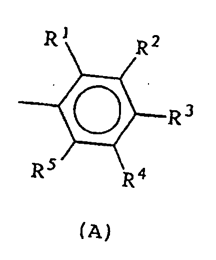 worin R¹ bis R⁵ jeweils unabhängig voneinander stehen für Wasserstoff, Fluor, Chlor, Methyl, Methoxy, Trifluormethyl, Trifluormethoxy, Cyano oder Nitro, worin einer oder mehrere der Reste R², R³, R⁴ und R⁵ für Wasserstoff stehen.

4. Verbindung der folgenden Fonnel 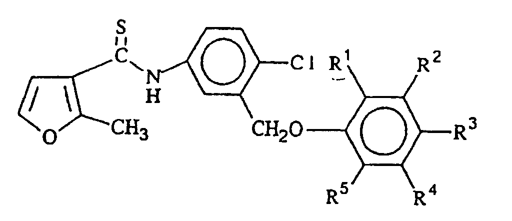 worin R¹ bis R⁵ jeweils unabhängig voneinander für Wasserstoff, Fluor, Chlor, Methyl, Methoxy, Trifluormethyl, Trifluormethoxy. Cyano oder Nitro stehen, wobei einer oder mehrere der Reste R², R³, R⁴ und R⁵ für Wasserstoff stehen.

5. Verbindung nach Anspruch 4, worin R¹ und R⁵ für Fluor steht und R², R³ und R⁴ für Wasserstoff stehen.

6. Verbindung nach Anspruch 4, worin R¹ für Nitro steht und R², R³, R⁴ und R⁵ für Wasserstoff stehen.

7. Verbindung nach Anspruch 4, worin R¹ für Fluor steht, R⁵ für Methoxy steht und R², R³ und R⁴ für Wasserstoff stehen.

8. Verbindung nach Anspruch 4, worin R¹ für Brom steht und R², R³, R⁴ und R⁵ für Wasserstoff stehen.

9. Verbindung nach Anspruch 4, worin R¹, R² und R⁵ für Fluor stehen und R³ und R⁴ für Wasserstoff stehen.

10. Verbindung nach Anspruch 4, worin R¹ für Trifluormethyl steht und R², R³, R⁴ und R⁵ für Wasserstoff stehen.

11. Verbindung nach Anspruch 4, worin R¹, R², R³ und R⁵ für Fluor stehen und R⁴ für Wasserstoff steht.

12. Verbindung nach Anspruch 4, worin R¹ für Fluor steht und R², R³, R⁴ und R⁵ für Wasserstoff stehen.

13. Verbindung der folgenden Fonnel

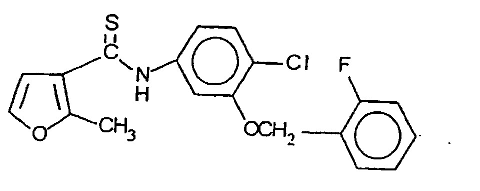

14. Pharmazeutische Zusammensetzung mit Eignung zur Behandlung einer HIV-1-Infektion bei einem infizierten Wirt, wobei die Zusammensetzung eine therapeutisch wirksame Menge der Verbindung nach Anspruch 1 und einen pharmazeutisch akzeptablen Träger umfasst.

15. Pharmazeutische Zusammensetzung mit Eignung zur Behandlung einer HIV-1-Infektion bei einem infizierten Wirt, wobei die Zusammensetzung eine therapeutisch wirksame Menge der Verbindung nach Anspruch 2 und einen pharmazeutisch akzeptablen Träger umfasst.

16. Pharmazeutische Zusammensetzung mit Eignung zur Behandlung einer HIV-1-Infektion bei einem infizierten Wirt, wobei die Zusammensetzung eine therapeutisch wirksame Menge der Verbindung nach Anspruch 3 und einen pharmazeutisch akzeptablen Träger umfasst.

17. Pharmazeutische Zusammensetzung mit Eignung zur Behandlung einer HIV-1-Infektion bei einem infizierten Wirt, wobei die Zusammensetzung eine therapeutisch wirksame Menge der Verbindung nach Anspruch 4 und einen pharmazeutisch akzeptablen Träger umfasst.

18. Pharmazeutische Zusammensetzung mit Eignung zur Behandlung einer HIV-1-Infektion bei einem infizierten Wirt, wobei die Zusammensetzung eine therapeutisch wirksame Menge der Verbindung nach Anspruch 13 und einen pharmazeutisch akzeptablen Träger umfasst.

19. Verwendung der Verbindung nach einem der Ansprüche 1 bis 4 und 13 zur Herstellung eines Medikaments zur Behandlung einer HIV-1-Infektion bei einem befallenen Wirt.

20. Verwendung einer Verbindung nach einem der Ansprüche 1 bis 4 und 13 zur Herstellung eines Medikaments zur Hemmung der Replikation von HIV-1.

## Revendications

1. Composé de formule 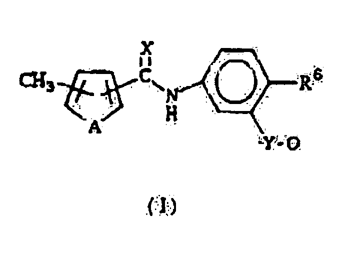 dans laquelle
A et X sont indépendamment de l'oxygène ou du soufre ;
R⁶ est H, un halogène, un alkyle en C₁-C₄, un alcoxy en C₁-C₄, un thio-, cyano-, ou nitroalkyle en C₁-C₄ ;
Y est -CH₂O-, -OCH₂-, -CH₂S- ou -CH₂SO₂- ;
Q est :
(A) un groupe de structure 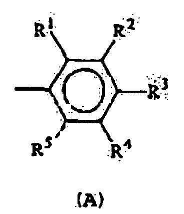 dans laquelle R¹ à R⁵ sont chacun indépendamment :
(i) un hydrogène, un halogène, un alkyle en C₁-C₆, un alcoxy en C₁-C₄, un thioalkyle en C₁-C₄, un haloalcoxy en C₁-C₄, un cyano, un nitro, un hydroxy, un acétyloxy, un benzoyloxy, un amino, un acétamido, un phényle, un acétyloxyméthyle, un hydroxyméthyle, un trihalogénométhyle, un carboxy, un alcoxycarbonyle en C₁-C₄, un formyle, un alkylcarbonyle en C₁-C₄, un benzoyle
ou (ii) un groupe de formule 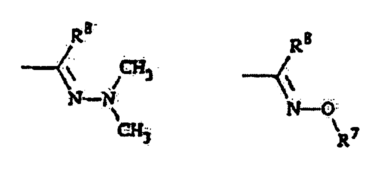 dans laquelle R⁷ est H, un alkyle en C₁-C₄ linéaire ou ramifié, un haloalkyle en C₁-C₄, un aminocarbonylméthyle, un alcoxycarbonylméthyle en C₂-C₆, un cyanométhyle ou un arylméthyle et R⁸ est un hydrogène ou un méthyle ;
ou (B) un groupe de formule 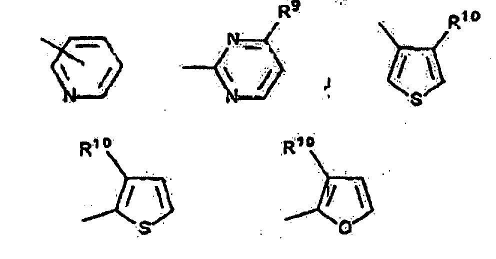 dans laquelle
R⁹ est un hydrogène, un alkyle en C₁-C₄ ou halogénoalkyle en C₁-C₄ et
R¹⁰ est H, un halogène, un alkyle en C₁-C₄ ou un alcoxycarbonyle en C₁-C₄.

2. Composé selon la revendication 1, dans lequel :
A est de l'oxygène ou du soufre ;
X est du soufre ;
R6 est un halogène, un alkyle en C₁-C₄, un alcoxy en C₁-C₄, un thio- ou cyanoalkyle en C₁-C₆- ;
Y est -CH₂O-, -OCH₂- ou -CH₂S- ;
Q est un groupe de structure 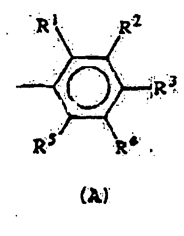
dans laquelle R¹ à R⁵ sont chacun indépendamment un hydrogène, un halogène, un alkyle en C₁-C₄, un alcoxy en C₁-C₆, un trihalogénométhyle, cyano, nitro ou trihalogénométhoxy.

3. Composé selon la revendication 2, dans lequel :
R⁶ est un halogène, un méthoxy ou un cyano ;
Y est -CH₂O-, -OCH₂- ;
Q est un groupe aromatique de structure 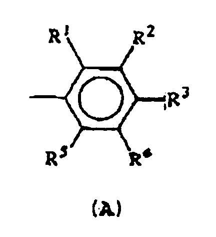
dans laquelle R¹ à R⁵ sont chacun indépendamment un hydrogène, un fluoro, un chloro, un méthyle, un méthoxy, un trifluorométhyle, un trifluorométhoxy, un cyano ou un nitro, dans lequel un ou plusieurs des groupements R², R³, R⁴ et R⁵ sont un hydrogène.

4. Composé de formule 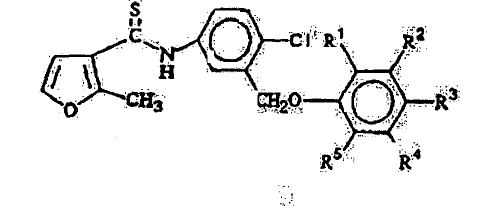 dans laquelle R¹ à R⁵ sont chacun indépendamment un hydrogène, un fluoro, un chloro, un méthyle, un méthoxy, un trifluorométhyle, un trifluorométhoxy, un cyano ou un nitro, dans lequel un ou plusieurs des groupements R², R³, R⁴ et R⁵ sont un hydrogène.

5. Composé selon la revendication 4, dans lequel R¹ et R⁵ sont un fluor et R², R³ et R⁴ sont un hydrogène.

6. Composé selon la revendication 4, dans lequel R¹ est un nitro et R², R³, R⁴ et R⁵ sont un hydrogène.

7. Composé selon la revendication 4, dans lequel R¹ est un fluoro, R⁵ est un méthoxy et R², R³ et R⁴ sont un hydrogène.

8. Composé selon la revendication 4, dans lequel R¹ est un bromo et R², R³, R⁴ et R⁵ sont un hydrogène.

9. Composé selon la revendication 4, dans lequel R¹, R² et R⁵ sont un fluoro et R³ et R⁴ sont un hydrogène.

10. Composé selon la revendication 4, dans lequel R¹ est un trifluorométhyle et R², R³, R⁴ et R⁵ sont un hydrogène.

11. Composé selon la revendication 4, dans lequel R¹, R², R³ et R⁵ sont un f luoro et R⁴ est un hydrogène.

12. Composé selon la revendication 4, dans lequel R¹ est un fluoro et R², R³, R⁴ et R⁵ sont un hydrogène.

13. Composé de formule

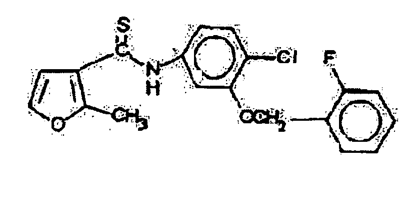

14. Composition pharmaceutique utile pour le traitement d'une infection à VIH-1 chez un hôte infecté, laquelle composition comprend une quantité efficace du point de vue thérapeutique du composé selon la revendication 1 et un véhicule pharmaceutiquement acceptable.

15. Composition pharmaceutique utile pour le traitement d'une infection à VIH-1 chez un hôte infecté, laquelle composition comprend une quantité efficace du point de vue thérapeutique du composé selon la revendication 2 et un véhicule pharmaceutiquement acceptable.

16. Composition pharmaceutique utile pour le traitement d'une infection à VIH-1 chez un hôte infecté, laquelle composition comprend une quantité efficace du point de vue thérapeutique du composé selon la revendication 3 et un véhicule pharmaceutiquement acceptable.

17. Composition pharmaceutique utile pour le traitement d'une infection à VIH-1 chez un hôte infecté, laquelle composition comprend une quantité efficace du point de vue thérapeutique du composé selon la revendication 4 et un véhicule pharmaceutiquement acceptable.

18. Composition pharmaceutique utile pour le traitement d'une infection à VIH-1 chez un hôte infecté, laquelle composition comprend une quantité efficace du point de vue thérapeutique du composé selon la revendication 13 et un véhicule pharmaceutiquement acceptable.

19. Utilisation du composé selon l'une quelconque des revendications 1 à 4 et 13 pour la fabrication d'un médicament destiné au traitement d'une infection à VIH-1 chez un hôte atteint.

20. Utilisation d'un composé selon l'une quelconque des revendications 1 à 4 et 13 pour la fabrication d'un médicament destiné à l'inhibition de la réplication du VIH-1.
